# EUROPEAN PATENT APPLICATION

(11) **EP 2 460 495 A1**
(43) Date of publication of application: **06.06.2012**
(21) Application number: 10193370.3
(22) Date of filing: 01.12.2010
(51) Int. Cl.: A61F 5/00

(54) **An endoluminal lining and a method for endoluminally lining a hollow organ**

(71) Applicant: Ethicon Endo-Surgery, Inc., Cincinnati, Ohio 45242-2839 (US)
(72) Inventor: Voegele, James W., Cincinnati, OH 45249 (US)
(74) Representative: Leihkauf, Steffen Falk

(57) **Abstract**

An endoluminal lining (1) for internally lining a hollow organ comprises a flexible tubular sleeve body (3), at least one anchoring portion (6) formed at an end (4) of the sleeve body and provided for the connection of the sleeve body (3) to the hollow organ, wherein the anchoring portion (6) comprises a tubular, longitudinally slit anchoring wall (7) elastically biased in a coiled compacted shape and deformable to an expanded substantially tubular shape for engaging the hollow organ (2), as well as locking means adapted to keep the anchoring wall (7) in the expanded shape.

## Description

### FIELD OF THE INVENTION

The present invention relates generally to medical apparatuses and methods and more particularly to devices and methods for internally lining a hollow body organ, such as a stomach, intestine or gastrointestinal tract.

### BACKGROUND OF THE INVENTION

ln cases of severe obesity, patients may currently undergo several types of surgery either to tie off or staple portions of the large or small intestine or stomach, and/or to bypass portions of the same to reduce the amount of food desired by the patient, and the amount absorbed by the gastrointestinal tract. The procedures currently available include laparoscopic banding, where a device is used to "tie off" or constrict a portion of the stomach, vertical banded gastroplasty (VBG), or a more invasive surgical procedure known as a Roux-En-Y gastric bypass to effect permanent surgical reduction of the stomach's volume and subsequent bypass of the intestine.

Although the outcome of these stomach reduction surgeries leads to patient weight loss because patients are physically forced to eat less due to the reduced size of their stomach, several limitations exist due to the invasiveness of the procedures, including time, general anesthesia, healing of the incisions and other complications attendant to major surgery. In addition, these procedures are only available to severely obese patients (morbid obesity, Body Mass Index > 40) due to their complications, including the risk of death, leaving patients who are considered obese or moderately obese with few, if any, interventional options.

In addition to the above described gastrointestinal reduction surgery, endoluminal sleeves are known for partially or totally lining certain portions of the stomach and of the intestine with the aim to separate or bypass at least part of the food flow from the lined portions of the gastrointestinal tract. It has been observed that by creating a physical barrier between the ingested food and certain regions of the gastrointestinal wall by means of endoluminal sleeves, similar benefits for weight loss and improvement or resolution of type 2 diabetes may be achieved as with gastric bypass surgery. Physicians believe that by creating a physical barrier between the ingested food and selected regions of the gastrointestinal wall, it might be possible to purposefully influence the mechanism of hormonal signal activation originating from the intestine.

A known type of endoluminal sleeve relies on metallic expandable structures, such as a stent, to engage the surrounding hollow organ for holding the sleeve in the planned position.

This notwithstanding, it has been observed that correct positioning and anchoring endoscopic sleeves is still complicated and the deployed sleeves tend to move inside the Gl tract and migrate away from their initially planned position. Furthermore, the known methods and devices for endoluminally lining hollow organs, particularly sections of the gastrointestinal tract, do not sufficiently address the needs and specific problems arising during transoral endoluminal deployment, removal, substitution or relocation of endoluminal sleeves.

Accordingly, there is a need for improved devices and procedures for deploying and removing endoluminal sleeves from the GI tract.

### SUMMARY OF THE INVENTION

The present invention provides for an improved lining and method for the transoral, or endoscopic, positioning, anchoring and removal of an endoluminal lining within a hollow body organ, particularly the gastrointestinal tract, including, but not limited to, the esophagus, stomach, portions of or the entire length of the intestinal tract, etc., unless specified otherwise. In the case of the present invention, the surgeon or endoscopist may insert devices as described below through the patient's mouth, down the esophagus and into the stomach or intestine as appropriate. The procedure can be performed entirely from within the patient's stomach or other intestinal tract, and does not necessarily require any external incision.

At least part of the above identified needs are met by an endoluminal lining for internally lining a hollow organ, particularly a section of the gastrointestinal tract, the lining comprising a flexible tubular sleeve body extending between a proximal end and a distal end, at least one anchoring portion formed at one of said proximal end and distal end, the anchoring portion being provided for the connection of the sleeve body to the hollow organ and comprises a tubular, longitudinally slit anchoring wall with two opposite longitudinal edges. The anchoring wall is elastically biased in a coiled compacted shape and deformable to an expanded substantially tubular shape to engage the hollow organ, wherein locking means are provided for keeping the anchoring wall in the expanded shape.

When the anchoring wall is in the coiled compacted configuration, the lining can be comfortably transorally transported to the target site in the GI tract and, after positioning of the anchoring portion in the desired place, the anchoring wall can be deformed to the expanded tubular shape to engage the lumen wall and anchor the lining thereto. The coil feature permits a great difference between the compacted bulk and the expanded dimension of the anchoring portion and reconciles the contrasting needs of a small bulk during endoluminal insertion and removal of the lining and a strongly expanded anchoring portion able to resist peristalsis and to avoid undesired dislocation.

In accordance with an aspect of the invention, the locking means include at least one locking seat formed in an internal surface of the anchoring wall and a corresponding locking ring which is displaceable from a rest position outside the locking seat, in which the locking ring allows the anchoring wall to rest in the compacted shape, and an activated position inside the locking seat, in which the locking ring prevents the anchoring wall from elastically returning to the compacted shape.

In accordance with an aspect of the invention, the locking ring itself is deformable from a collapsed shape to an enlarged shape and configured such that, when the anchoring wall is in the compacted shape and the locking ring is in the rest position, the anchoring wall elastically holds the locking ring in its collapsed shape and, when the anchoring wall is in the expanded shape and the locking ring is in the activated position, the locking ring is released to take its enlarged shape and holds the anchoring wall in its expanded shape. For this purpose, the locking ring is preferably manufactured to be permanently elastically biased in the enlarged shape.

In accordance with a further aspect of the invention, the longitudinal edge sections of the anchoring wall are adapted to connect each other by shape coupling when the anchoring wall is deformed to its expanded shape and to prevent the anchoring wall from elastically relaxing to the compacted shape.

In accordance with a further aspect of the invention, the anchoring wall defines an internal sleeve seat adapted to receive and hold the flexible sleeve body in a longitudinally compacted, wrapped, folded or rolled shape and to release the sleeve body during the deformation of the anchoring wall from the compacted shape to the expanded shape, so that the sleeve body can be unfolded distally into an elongate tubular shape.

At least part of the above identified needs are further met by a method for applying and removing a tubular lining from a hollow organ, particularly to a section of the gastrointestinal tract, comprising the steps of:
- providing a lining having a flexible tubular sleeve body, an anchoring portion comprising an anchoring wall elastically biased in a coiled compacted shape and deformable to an expanded substantially tubular shape to engage the hollow organ, and locking means for keeping the anchoring wall in the expanded shape,
- inserting the lining with the compacted coiled anchoring wall endoluminally in the hollow organ, and
- fixating the anchoring portion of the lining to the wall of the hollow organ by deforming the coiled anchoring wall from the compacted shape to the tubular expanded shape and displacing the locking means from a rest position to an activated position, in which the locking means prevent the anchoring wall from returning to the compacted shape.

In accordance with an aspect of the invention, after a period of time, the lining is detached from the hollow organ wall by endoluminally accessing the lining near the anchoring portion, removing the locking means from the activated position to allow the anchoring wall to deform back to the coiled compacted shape.

In accordance with a further aspect of the invention, the step of deploying the lining in the hollow organ comprises inserting a balloon dilator in the anchoring portion of the lining, attaching the compacted lining together with the balloon dilator to an applier, endoluminally placing the applier with the attached lining and balloon dilator, using an endoscope to obtain visibility of an area suitable for the anchoring of the lining and deforming the coiled anchoring wall from the compacted shape to the tubular expanded shape by insufflating the balloon dilator inside the anchoring portion.

In accordance with an aspect of the invention, the method comprises the steps of housing a tubular sleeve body of the lining inside the compacted coiled anchoring wall in a collapsed, e.g. wrapped, folded, compressed or rolled up configuration with regard to a lining longitudinal extension and, after fastening the anchoring wall to the hollow organ, pulling the tubular sleeve body to unfold it from the collapsed configuration to an extended elongate tubular shape.

These and other aspects and advantages of the present invention shall be made apparent from the accompanying drawings and the description thereof, which illustrate embodiments of the invention and, together with the general description of the invention given above, and the detailed description of the embodiments given below, serve to explain the principles of the present invention.

### DESCRIPTION OF THE DRAWINGS

- Figures 1 and 2 illustrate a longitudinal section of an endoluminal lining in accordance with an embodiment in a compacted configuration and in an expanded configuration within a section of the Gl tract (dashed line);
- Figure 3 is a schematic top view of an anchoring wall of the lining in figure 1 in a coiled compacted configuration;
- Figure 4 is a schematic top view of the anchoring wall of the lining in figure 2 in a tubular expanded configuration;
- Figures 5 and 6 illustrate opposite longitudinal edge regions of an anchoring wall of a lining in accordance with an embodiment in an overlapping configuration and in a mutually butted configuration;
- figure 7 is a partial side view of an endoluminal instrument for the deployment and removal or displacement of the lining within a hollow organ;

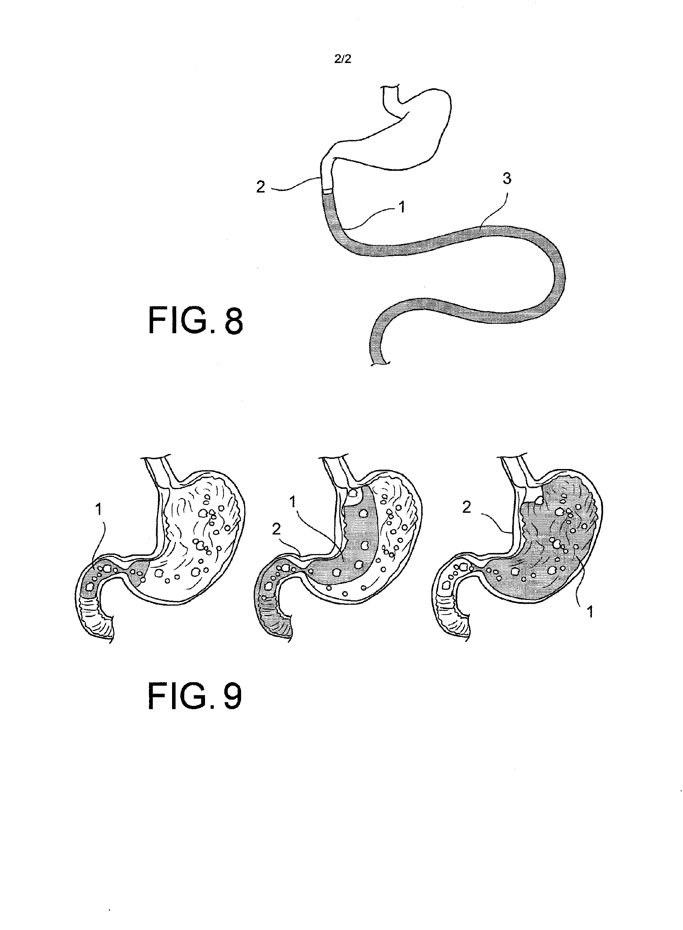

- Figure 8 illustrates the tubular lining after anchoring and full extension within a section of the GI tract;
- Figure 9 illustrates different possible positions of the tubular lining within the GI tract of a patient.

### DETAILED DESCRIPTION OF EMBODIMENTS

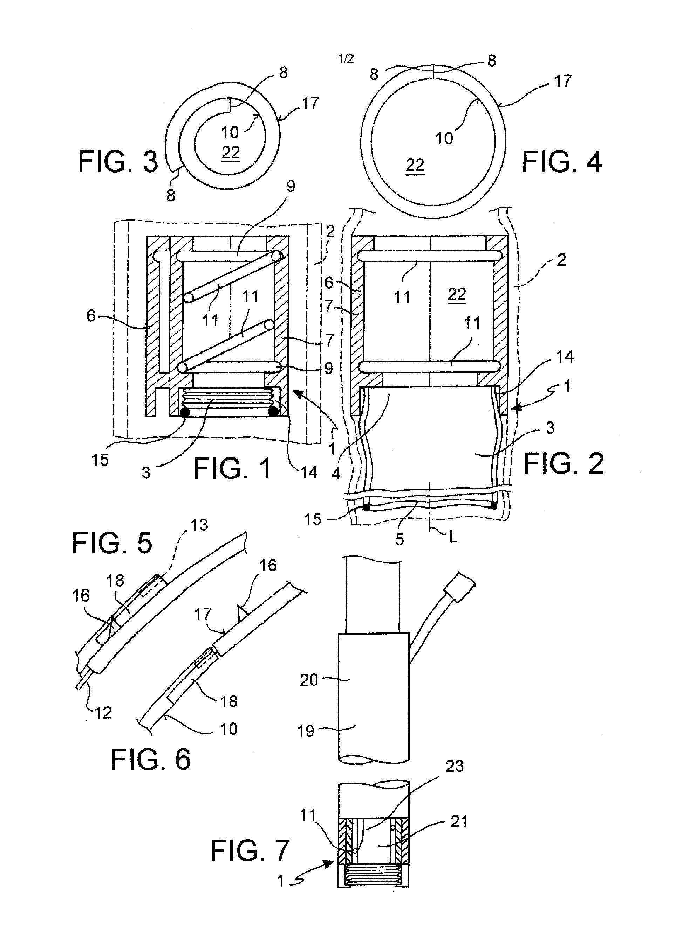

Referring to the drawings where like numerals denote like anatomical structures and components throughout the several views, figures 1 to 6 depict an endoluminal lining 1 for internally lining a hollow organ, particularly a section of the gastrointestinal tract 2. The lining 1 comprises a flexible tubular sleeve body 3 extending between a proximal end 4 and a distal end 5 and at least one anchoring portion 6 formed at one of said proximal end 4 and distal end 5. The anchoring portion 6 is provided for the connection of the sleeve body 3 to the hollow organ. The anchoring portion 6 comprises a tubular, longitudinally slit anchoring wall 7 with two opposite longitudinal edges 8. The anchoring wall 7 is elastically biased in a coiled compacted shape (Figures 1 and 3) and deformable to an expanded substantially tubular shape (Figures 2 and 4) to engage the hollow organ 2, wherein locking means are provided for keeping the anchoring wall 7 in the expanded shape.

When the anchoring wall 7 is in the coiled compacted configuration, the lining 1 can be comfortably transorally transported to the target site in the GI tract and, after positioning of the anchoring portion 6 in the desired place, the anchoring wall 7 can be deformed to the expanded tubular shape to engage the lumen wall and anchor the lining 1 thereto. In this context, the coil feature permits a great difference between the compacted bulk and the expanded dimension of the anchoring portion and reconciles the contrasting needs of a small bulk during endoluminal insertion and removal of the lining and a strongly expanded anchoring portion able to resist peristalsis and to avoid undesired dislocation of the lining within the GI tract.

The anchoring wall 7 may be fabricated of elastic or super-elastic metal sheet material or plastic sheet material which has undergone a treatment to result permanently elastically biased in the compacted coiled shape and deformable, against the elastic pre-stress, into the expanded tubular shape.

In accordance with embodiments, the opposite longitudinal edges 8 of the expanded anchoring wall 7 may be frontally abutted against each other without overlap or, alternatively, the longitudinal edge regions of the anchoring wall 7 are still at least partially overlapping in a spiral manner.

In accordance with an embodiment, the above said locking means include at least one locking seat 9 formed in an internal (or in other words concave) surface 10 of the anchoring wall 7 and a corresponding locking ring 11. The locking ring 11 is displaceable from a rest position (figure 1) outside the locking seat 9, in which the locking ring 11 allows the anchoring wall 7 to rest in the compacted shape, and an activated position (figure 2) inside the locking seat 9, in which the locking ring 11 prevents the anchoring wall 7 from elastically returning to the compacted shape. The locking seat 9 may comprise a groove formed in the internal surface 10 and extending transversally and possibly perpendicularly to a longitudinal direction L of the anchoring portion 6. In this way a substantially ring shaped groove is provided which receives the locking ring with an approximately uniform radial preload.

The exemplary embodiment of figures 1 and 2 show a lining with two locking seats 9 and corresponding locking rings 11.

The locking ring 11 itself may be deformable from a, e.g. coiled, spiral, helix, ovalized or snap through arched, collapsed shape to an enlarged ring shape or ring section shape and configured such that, when the anchoring wall 7 is relaxed in the compacted shape and the locking ring 11 is in the rest position, the anchoring wall 7 elastically holds the locking ring 11 in its collapsed shape and, when the anchoring wall 7 is in the expanded shape and the locking ring 11 is in the activated position, the locking ring 11 relaxes to take its enlarged shape and holds the anchoring wall 7 in its expanded shape. For this purpose, the locking ring 11 is manufactured and treated to result permanently elastically preloaded into the enlarged shape.

In accordance with an exemplary embodiment (Figures 5 and 6), the longitudinal edge sections 8 of the anchoring wall 7 are adapted to connect each other by shape coupling when the anchoring wall 7 is deformed to its expanded shape and to prevent the anchoring wall 7 from elastically relaxing to the coiled compacted shape. For this purpose, the longitudinal edge sections 8 may form hook and latch connecting portions, e.g. of the tongue and groove type 12, 13.

In accordance with a further embodiment, the anchoring wall 7 defines an internal sleeve seat 14 adapted to receive and hold the flexible sleeve body 3 in a longitudinally compacted, wrapped, folded or rolled shape and to release the sleeve body 3 when the anchoring wall 7 is expanded, so that the sleeve body 3 can be unfolded distally into an elongate tubular shape.

The sleeve seat 14 may be defined by the internal surface 10 in a distal end region of the anchoring wall 7 and configured to elastically clamp and compress the sleeve body 3 in its longitudinally compacted shape when the anchoring wall 7 is relaxed in the compacted coiled shape. The proximal end 4 of the sleeve body may be directly permanently connected with the sleeve seat 14 or with one of the locking rings 11.

An inertial mass 15 may be provided at the distal end 5 of the sleeve body 3 to help distal unfolding the latter within the GI tract. The lining 1 may be formed of any suitable biocompatible graft material such as polyester or PTFE, rubber, Teflon, Nylon, Dacron, polyethylene, polystyrene, polyurethane, polyethylene terephtalate, etc.

In accordance with a further embodiment, one or more radially outward protruding barbs 16 or hooks may be provided at an external surface 17 of the anchoring wall 7 to penetrate the lumen wall and prevent the lining from migrating away from its planned position. The barbs 16 are formed in a region of the external surface 17 which is covered by the anchoring wall 7 itself when the latter is relaxed in the compacted coiled configuration and which is exposed outwardly when the anchoring wall 7 is expanded. In order to avoid damage of the barbs 16 and friction between the barbs 16 and the anchoring wall during expansion and coiling, the internal surface 10 may form guide grooves 18 which open in the longitudinal edge 8 and receive and protect the barbs 16 when the anchoring wall takes the compacted coiled shape.

Figure 7 illustrates an exemplary endoluminal applier 19 for the deployment and removal of the lining 1 from the hollow organ. The applier 19 has a flexible shaft 20 and a dilating tip 21, e.g. a balloon dilator tip connected to a preferably extracorporeal fluid pump (not illustrated). The dilating tip 22 is configured to be insertable inside an internal space 22 defined by the anchoring wall 7 and connectable to the anchoring wall by an elastic interference fit. The dilating tip 22 is further adapted to expand the anchoring wall from the compacted shape to the expanded shape which is then maintained by the above described locking means, and from the expanded shape to a slightly more expanded shape which allows the locking means to be released and the anchoring wall to relax to the coiled compacted shape.

The locking rings 11 may be moved into and out of the corresponding locking seats 9 by the dilating tip 21 or by additional manipulation means 23 of the applier 19, such as e.g. pulling strings or push portions, which are connected with or adapted to act on the locking rings 11.

In this way a bypass conduit may be purposefully created and removed to achieve a malabsorptive effect in cases where such an effect may enhance weight loss, as well as the initially described effects on hormonal signaling in general. Particularly, the described procedures and devices help to mimic the effects of gastric bypass in resolution of type 2 diabetes and facilitating weight loss, improve glycemic control and reduce or eliminate other co-morbidities of severe obesity. Moreover, the described procedures and devices may be advantageously used in conjunction with other therapeutic regimes for the treatment of type 2 diabetes and its co-morbidities and address the patients fear of invasive surgery. Last but not least, the described procedures and devices allow a reversible procedure with an easy removal and replacement of the endoluminal sleeve once the desired effect has been achieved or a modification of the endoluminal lining is desired.

Although preferred embodiments of the invention have been described in detail, it is not the intention of the applicant to limit the scope of the claims to such particular embodiments, but to cover all modifications and alternative constructions falling within the scope of the invention.

## Claims

**1.** An endoluminal lining (1) for internally lining a hollow organ, particularly a section of the gastrointestinal tract (2), the lining (1) comprising:
- a flexible tubular sleeve body (3) extending between a proximal end (4) and a distal end (5),
- at least one anchoring portion (6) provided at one of said proximal end (4) and distal end (5) for the connection of the sleeve body (3) to the hollow organ, wherein said anchoring portion (6) comprises:
- a tubular, longitudinally slit anchoring wall (7) elastically biased in a coiled compacted shape and deformable to an expanded substantially tubular shape for engaging the hollow organ (2), and
- locking means adapted to keep the anchoring wall (7) in the expanded shape.

**2.** An endoluminal lining (1) according to claim 1, in which said anchoring wall (7) comprises an elastic metal sheet which has undergone a treatment to become permanently elastically biased in the compacted coiled shape and deformable, against the elastic pre-stress, into the expanded tubular shape.

**3.** An endoluminal lining (1) according to claim 1 or 2, in which opposite longitudinal edges (8) of the anchoring wall (7) are frontally abutted against each other when the anchoring wall is locked in the expanded shape.

**4.** An endoluminal lining (1) according to any one of the preceding claims, wherein said locking means include at least one locking seat (9) formed in an internal surface (10) of the anchoring wall (7) and at least one corresponding locking ring (11) displaceable from a rest position outside the locking seat (9), in which the locking ring (11) allows the anchoring wall (7) to rest in the compacted shape, and an activated position inside the locking seat (9), in which the locking ring (11) prevents the anchoring wall (7) from elastically returning to the compacted shape.

**5.** An endoluminal lining (1) according to claim 4, wherein the locking seat (9) comprises a groove formed in the internal surface (10) and extending substantially perpendicularly to a longitudinal direction (L) of the anchoring portion (6) and forms a substantially ring shaped locking seat (9).

**6.** An endoluminal lining (1) according to claims 4 and 5, in which the anchoring portion (6) comprises two locking seats (9) and two corresponding locking rings (11).

**7.** An endoluminal lining (1) according to any one of claims 4 to 6, in which the locking ring (11) itself is deformable from a collapsed shape to an enlarged shape and configured such that:
- when the anchoring wall (7) is relaxed in the compacted shape and the locking ring (11) is in the rest position, the anchoring wall (7) elastically holds the locking ring (11) in its collapsed shape, and
- when the anchoring wall (7) is in the expanded shape and the locking ring 11 is in the activated position, the locking ring (11) relaxes to take its enlarged shape and holds the anchoring wall (7) in its expanded shape.

**8.** An endoluminal lining (1) according to any one of claims 4 to 7, in which the locking ring (11) is manufactured to be permanently elastically preloaded into the enlarged shape.

**9.** An endoluminal lining (1) according to any one of claims 4 to 8, in which said collapsed shape of the locking ring (11) is a coiled, spiral, helix, ovalized or snap through arched shape.

**10.** An endoluminal lining (1) according to any one of the preceding claims, in which the anchoring wall (7) forms opposite longitudinal edge sections (8) adapted to connect to each other by shape coupling when the anchoring wall (7) is deformed to its expanded shape, thereby preventing the anchoring wall (7) from elastically relaxing back to the coiled compacted shape. **11**. An endoluminal lining (1) according to claim 10, in which said longitudinal edge sections (8) form a tongue and groove (12, 13) system.

**12.** An endoluminal lining (1) according to any one of the preceding claims, in which said anchoring wall (7) defines an internal sleeve seat (14) adapted to receive and hold the flexible sleeve body (3) in a compacted shape and to release the sleeve body (3) to unfold when the anchoring wall (7) is expanded.

**13.** An endoluminal lining (1) according to claim 12, in which said sleeve seat (14) is configured to elastically clamp and compress the sleeve body (3) in its longitudinally compacted shape when the anchoring wall (7) is relaxed in the compacted coiled shape.

**14.** An endoluminal lining (1) according to any one of the preceding claims, comprising at least one radially outward protruding barb (16) provided in a region of an external surface (17) of the anchoring wall (7) which is covered by the anchoring wall (7) in the compacted coiled configuration and exposed outwardly when the anchoring wall (7) is expanded.

**15.** An endoluminal lining (1) according to claim 14, in which an internal surface (10) of the anchoring wall (7) forms guide grooves (18) which open in a longitudinal edge (8) and receive and protect the barbs (16) when the anchoring wall (7) takes the compacted coiled shape.

**16.** Endoluminal lining system, including an endoluminal lining (1) according to any one of the preceding claims and an endoluminal applier (19) for the deployment and removal of the lining (1) from the hollow organ, said applier (19) comprising:
- a flexible shaft (20),
- a dilating tip (21) formed at a distal end of the flexible shaft (20) and insertable inside an internal space (22) defined by the anchoring wall (7) and connectable to the anchoring wall by an elastic interference fit, in which the dilating tip (21) is adapted to expand the anchoring wall from the compacted shape to the expanded shape and from the expanded shape to a slightly more expanded shape which allows the locking means to be released and the anchoring wall to relax to the coiled compacted shape,
- manipulation means (23) adapted to act on the locking means (11) of the anchoring wall (7).

**17.** A method for lining from a hollow organ, particularly to a section of the gastrointestinal tract, the method comprising the steps of:
- providing a lining having a flexible tubular sleeve body, an anchoring portion forming an anchoring wall elastically biased in a coiled compacted shape and deformable to an expanded substantially tubular shape to engage the hollow organ, and locking means for keeping the anchoring wall in the expanded shape,
- inserting the lining with the compacted coiled anchoring wall endoluminally in the hollow organ, and
- fixating the anchoring portion of the lining to the wall of the hollow organ by deforming the coiled anchoring wall from the compacted shape to the tubular expanded shape and displacing the locking means from a rest position to an activated position, in which the locking means prevent the anchoring wall from returning to the compacted shape.

**18.** A method according to claim 17, in which, after a period of time, the lining is detached from the hollow organ wall by endoluminally accessing the lining near the anchoring portion, removing the locking means from the activated position to allow the anchoring wall to deform back to the coiled compacted shape.

**19.** A method according to claim 17 or 18, in which the step of anchoring the lining in the hollow organ comprises:
- inserting a balloon dilator in the anchoring portion of the lining,
- attaching the compacted lining together with the balloon dilator to an applier,
- endoluminally placing the applier with the attached lining and balloon dilator,
- using an endoscope to obtain visibility of an area suitable for the anchoring of the lining, and
- deforming the coiled anchoring wall from the compacted shape to the tubular expanded shape by insufflating the balloon dilator inside the anchoring portion.

**20.** A method according to claim 17, comprising:
- housing a tubular sleeve body of the lining inside the compacted coiled anchoring wall in a collapsed shape with regard to a lining longitudinal extension, and
- after fastening the anchoring wall to the hollow organ, pulling the tubular sleeve body to unfold it from the collapsed shape to an extended elongate tubular shape.
